Europäisches Patentamt

European Patent Office

Office européen des brevets

(19)

(11) Veröffentlichungsnummer: **0 077 515**
**B1**

(12) EUROPÄISCHE PATENTSCHRIFT

(45) Veröffentlichungstag der Patentschrift:
20.03.85

(21) Anmeldenummer: 82109382.0

(22) Anmeldetag: 11.10.82

(51) Int. Cl.⁴: **G 01 N 33/72**

(54) Verfahren und Reagenz zur Bestimmung von glykosiliertem Hämoglobin.

(30) Priorität: 16.10.81 DE 3141146

(43) Veröffentlichungstag der Anmeldung:
27.04.83 Patentblatt 83/17

(45) Bekanntmachung des Hinweises auf die Patenterteilung:
20.03.85 Patentblatt 85/12

(84) Benannte Vertragsstaaten:
AT BE CH DE FR GB IT LI LU NL SE

(56) Entgegenhaltungen:
DE - A - 2 950 457
DE - A - 3 016 555
US - A - 3 350 174

IMMUNOCHEMISTRY, Band 8, 1971, Tokyo T. SASAZUKI
"Immunochemical and physicochemical properties of haptoglobin, antihemoglobin antibody, and their complexes with hemoglobin" Seiten 695-704
THE JOURNAL BIOLOGICAL CHEMISTRY, Band 242, Nr. 15, 17. Januar 1967, New York R.L. NAGEL et al.
"Kinetics and mechanism of complex formation between hemoglobin and haptoglobin" Seiten 3428-3434

(73) Patentinhaber: Boehringer Mannheim GmbH,
Sandhoferstrasse 116, D-6800 Mannheim 31 (DE)

(72) Erfinder: Deeg, Rolf, Dr.rer.nat., Seeseitener Strasse 18,
D-8124 Seeshaupt (DE)
Erfinder: Schmitt, Urban, Kustermannstrasse 29,
D-8132 Tutzing (DE)
Erfinder: Ziegenhorn, Joachim, Dr.rer.nat.,
Ina-Seidel-Weg 1, Starnberg (DE)

ACTORUM AG

## Beschreibung

Die Erfindung bezieht sich auf ein Verfahren zur Bestimmung von glykosiliertem Hämoglobin in Blutproben.

Glykosiliertes Hämoglobin (HbA$_1$) ensteht durch nicht enzymatische Glykosilierung des Hämoglobins (HbA$_o$). Normalerweise beträgt die Konzentration an glykosiliertem Hämoglobin in Blut etwa 5%, bezogen auf das Gesamthämoglobin. Bei Diabetikern ist diese Konzentration auf das 2- bis 4fache erhöht.

Die Bestimmung des glykosilierten Anteils am Gesamthämoglobin hat in den letzten Jahren Bedeutung bei der Diabetes-Diagnostik erlangt (vgl. L. Jovanovis und C.M. Peterson, „Am. J. Med." 70 [1981], S. 331-338). Die Reaktion zwischen einzelnen Hämoglobinmolekülen und der Glukose ergibt ein stabiles Reaktionsprodukt, das während der gesamten Lebenszeit der Erythrozyten, also ca. 100 bis 200 d, erhalten bleibt. Kurzzeitige Schwankungen des Blutzuckergehaltes beeinflussen die Konzentration an glykosiliertem Hämoglobin nicht entscheidend. Die Konzentration an glykosiliertem Hämoglobin spiegelt daher relativ genau die durchschnittliche Glukosekonzentration im Blut eines Patienten über einen langen Zeitraum hinweg wieder.

Es sind mehrere Verfahren zur Bestimmung des glykosilierten Anteils am Gesamthämoglobin bekannt. In klinischen Labors am weitesten verbreitet sind chromatographische Trennverfahren. Hierbei wird der glykosilierte Anteil des Hämoglobins vom nicht glykosilierten mit Hilfe einer Chromatographiesäule, einer Mikrosäule oder auch mit Hilfe der HPLC-Methode (HPLC = *high pressure liquid chromatography*) getrennt, wobei die Säulen mit einem Ionenaustauscher, beispielsweise mit Bio-Rex 70 [R] gefüllt sind. All diese Methoden sind jedoch sehr empfindlich gegen Änderungen des pH-Wertes, der Temperatur, der Ionenkonzentrationen. Die Trennverfahren müssen daher sehr vorsichtig durchgeführt werden, um optimale Resultate zu erhalten (vgl. a. a. O. S. 332).

Aus DE-A Nr. 2950457 ist ein Verfahren zur Bestimmung des glykosilierten Hämoglobins in Blutproben bekannt, bei dem die Veränderung der spektroskopischen Eigenschaften einer Blutprobe bei Zugabe eines allosterischen Effektors für die Bestimmung des HbA$_1$ ausgenutzt wird. Beeinflusst wird hierbei lediglich der nicht glykosilierte Hauptanteil des Hämoglobins. Die gemessenen Extinktionsdifferenzen sind im relevanten Bereich sehr klein. Sie werden ausserdem noch geringer, wenn der glykosilierte Anteil am Gesamthämoglobin zunimmt.

Aufgabe der Erfindung war es daher, ein Verfahren bereitzustellen, mit dem das glykosilierte Hämoglobin auf schnelle, technisch einfach durchführbare Weise zuverlässig und genau bestimmt werden kann.

Gelöst wird diese Aufgabe durch das erfindungsgemässe Verfahren, bei dem nach einer chemischen oder physikalischen Behandlung der Blutprobe zur Freisetzung des Hämoglobins aus den Erythrozyten eine Differenzierung des glykosilierten und nicht glykosilierten Hämoglobinanteils mit Hilfe von Haptoglobin durchgeführt und entweder die Reaktion zwischen Hämoglobin und Haptoglobin kinetisch verfolgt oder der an Haptoglobin gebundene bzw. der von Haptoglobin nicht gebundene Anteil des Hämoglobins nach bekannten Methoden gemessen wird. Unter Differenzierung von glykosiliertem und nicht glykosiliertem Hämoglobin werden sämtliche Methoden verstanden, die eine Unterscheidung des glykosilierten und nicht glykosilierten Hämoglobins aufgrund deren chemischen und physikalischen Eigenschaften zulassen.

Gegenstand der vorliegenden Erfindung ist somit ein Verfahren zur Bestimmung von glykosiliertem Hämoglobin in Blutproben, wobei zunächst durch chemische oder physikalische Behandlung der Blutprobe glykosiliertes und nicht glykosiliertes Hämoglobin aus den Erythrozyten freigesetzt, gegebenenfalls das Hämoglobin in Methämoglobin übergeführt, danach eine Differenzierung von glykosiliertem und nicht glykosiliertem Hämoglobin durchgeführt und anschliessend der nicht glykosilierte oder auch glykosilierte Anteil des Hämoglobins in bekannter Weise bestimmt wird, dadurch gekennzeichnet, dass die Differenzierung von glykosiliertem und nicht glykosiliertem Hämoglobin mit Hilfe von Haptoglobin vorgenommen wird.

Das erfindungsgemässe Verfahren kann in Gegenwart eines geeigneten Puffersystems durchgeführt werden. Als geeignetes Puffersystem kann im Rahmen der Erfindung jeder im pH-Bereich von 4,0 bis 8,5, vorzugsweise von 6,0 bis 7,0 wirksame Puffer verwendet werden. Besonders bewährt haben sich Phosphat- und Bis-Tris-Puffer.

Haptoglobin (Hp) ist ein Plasmaprotein. Es ist seit langem bekannt, dass Haptoglobin aus den Erythrozyten freigesetztes Hämoglobin zu binden vermag (s. hierzu T. Sasazuki, „Immunochemistry", 8 [1971], S. 695-704).

Es konnte nun überraschenderweise festgestellt werden, dass sich glykosiliertes und nicht glykosiliertes Hämoglobin hinsichtlich ihres Bindungsverhaltens gegenüber Haptoglobin unterscheiden. Das glykosilierte Hämoglobin wird rascher an das Haptoglobin gebunden als das nicht glykosilierte Hämoglobin. Dies folgt beispielsweise aus der Abnahme der Fluoreszenzintensität einer Haptoglobinenthaltenden Messlösung nach Zusatz von glykosiliertem bzw. nicht glykosiliertem Hämoglobin. Die Messung der Fluoreszenzstrahlung, die ein Mass für die Wechselwirkung zwischen Hämoglobin und Haptoglobin darstellt, kann in bekannter Weise erfolgen.

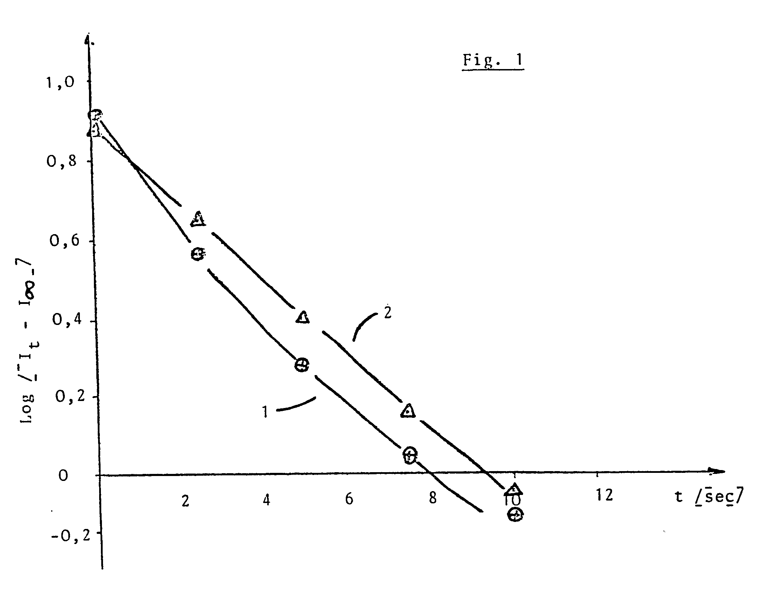

In Fig. 1 ist die Abnahme der Fluoreszenzintensität für beide Messlösungen gegen die Zeit aufgetragen. Es ist ersichtlich, dass die Fluoreszenzintensität nach Zugabe von glykosiliertem Hämoglobin schneller abnimmt als nach Zugabe von nicht glykosiliertem Hämoglobin. Dies deutet darauf hin, dass die Bindungswirkung zwischen Haptoglobin und dem glykosilierten Hämoglobin stärker ist als zwischen Haptoglobin und dem nicht glykosilierten Anteil.

Das Verfahren zur Bestimmung des glykosilierten Anteils am Gesamthämoglobin wird zweckmässigerweise derart durchgeführt, dass man zunächst nach üblichen Methoden aus den Erythrozyten das glykosilierte und das nicht glykosilierte Hämoglobin freisetzt. Der hämolysierten Blutprobe wird, gegebenenfalls nach vorherigem Zusatz eines geeigneten Oxidationsmittels zur Überführung des Hämoglobins in das Methämoglobin, eine Haptoglobin enthaltende Lösung zugegeben. Der glykosilierte Anteil des Hämoglobins wird bevorzugt an das Haptoglobin gebunden. Zur Unterscheidung des gebundenen vom nicht gebundenen Anteil des Hämoglobins bzw. Methämoglobins werden übliche Messmethoden eingesetzt. Durch Messen verschiedener hämoglobinhaltiger Proben mit unterschiedlichem glykosiliertem Hämoglobingehalt kann eine Eichkurve aufgestellt werden, anhand derer der glykosilierte Anteil an Gesamthämoglobin in unbekannten Proben ermittelt werden kann.

Der Unterschied im Bindungsverhalten von glykosiliertem und nicht glykosiliertem Hämoglobin gegenüber Haptoglobin kann durch Zusatz einer oder mehrerer Verbindungen mit Bindungswirkung auf die allosterische Effektorstelle verstärkt werden. Solche Verbindungen sind bekannt. Beispielhaft seien erwähnt: organische Phosphorverbindungen, wie 2,3-Diphosphoglycerat und Inosithexaphosphat, organische Sulfate, wie Inosithexasulfat, und Carbonsäuren, wie Mellitsäure.

Nach einer bevorzugten Ausführungsform des erfindungsgemässen Verfahrens wird somit die Differenzierung des glykosilierten und nicht glykosilierten Hämoglobins mit Hilfe von Haptoglobin unter Zusatz einer oder mehrerer Verbindungen mit Bindungswirkung auf die allosterische Effektorstelle des Hämoglobins durchgeführt. Zur Durchführung des erfindungsgemässen Verfahrens stehen dem Fachmann eine Reihe von Substanzen mit Bindungswirkung auf die allosterische Effektorstelle zur Verfügung. Im Sinne der Erfindung besonders geeignet sind Inosithexaphosphat, 2,3-Diphosphoglycerat und Mellitsäure. Sie werden der Lösung in mindestens äquivalenter Menge bezogen auf den Hämoglobingehalt zugegeben. Im allgemeinen ist es jedoch von Vorteil, diese Substanzen im Überschuss, vorzugsweise in 10- bis 200fachem molarem Überschuss einzusetzten.

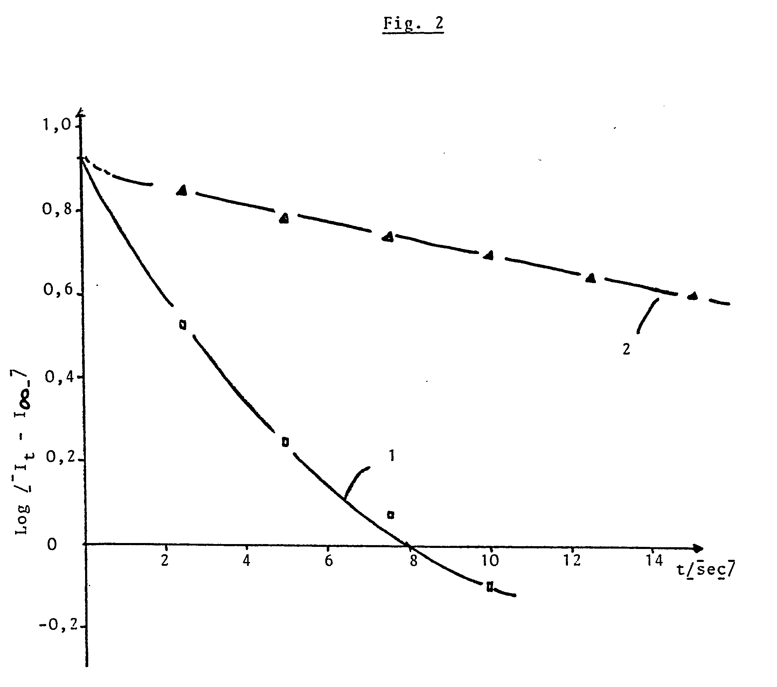

Der Einfluss von Inosithexaphosphat auf das Bindungsverhalten von glykosiliertem und nicht glykosiliertem Hämoglobin gegenüber Haptoglobin wird aus Fig. 2 deutlich. Hier ist die Abnahme der Fluoreszenzintensität einer Haptoglobinenthaltenden Lösung nach Zugabe von Inosithexaphosphat und nicht glykosiliertem Hämoglobin gegen die Zeit aufgetragen. Der Verlauf der Messkurven zeigt deutlich, dass in Gegenwart von Inosithexaphosphat glykosiliertes Hämoglobin wesentlich rascher an Haptoglobin gebunden wird als das nicht glykosilierte Hämoglobin.

Gegebenenfalls ist es zweckmässig, vor der Differenzierung von glykosiliertem und nicht glykosiliertem Hämoglobin das normalerweise nach der Hämolyse der Erythrozyten vorliegende Hämoglobin in das Methämoglobin überzuführen. Dem Fachmann stehen hierzu eine Reihe von bekannten Methoden zur Verfügung, beispielsweise Oxidation mit Kaliumhexacyanoferrat(III) oder mit Natriumnitrit.

Es ist von Vorteil, sowohl das Hämoglobin als auch gegebenenfalls das Methämoglobin mit einem oder mehreren hämbindenden Liganden zu stabilisieren. Prinzipiell eignen sich alle möglichen hämbindenden Liganden für das erfindungsgemässe Verfahren. Besonders bevorzugt sind Alkylisocyanide, Sauerstoff, Kohlenmonoxid und Stickstoffmonoxid als hämbindende Liganden für das Hämoglobin sowie Fluorid, Azid, Cyanid und Wasser als hämbindende Liganden für das Methämoglobin. Die Konzentration an hämbindenden Liganden in der Messlösung muss mindestens äquimolar zur Hämkonzentration sein. Da jedes Hämoglobinmolekül vier Hämreste aufweist, muss diese Minimalkonzentration der vierfachen Hämoglobinkonzentration entsprechen. Vorteilhaft werden jedoch auch die hämbindenden Liganden in grossem, vorzugsweise in 10- bis $10^5$fachem molarem Überschuss verwendet.

Ferner ist es zweckmässig, der Messlösung vor dem Kontaktieren mit Haptoglobin ein ionenbindungenstabilisierendes Mittel zuzusetzen. Solche Substanzen mit einer stabilisierenden Wirkung auf ionische Bindung sind beispielsweise Polyethylenglykole und Saccharose.

Hämoglobin kommt im Blut in zwei Formen vor: der Oxy- und der Deoxyform. Für das erfindungsgemässe Verfahren ist es zweckmässig, das in der hämolysierten Blutprobe enthaltene Hämoglobin in eine einheitliche Form zu bringen. Dies kann sowohl die Oxy- als auch die Deoxyform sein. Vorzugsweise wird der gesamte Hämoglobingehalt vor der Differenzierung des glykosilierten und nicht glykosilierten Hämoglobins in die Deoxyform übergeführt. Methoden zur Umwandlung der Oxy- in die Deoxyform bzw. der Deoxy- in die Oxyform sind dem Fachmann geläufig. Beispielsweise lässt sich die Oxyform mit Hilfe von Dithionit oder anderen Reduktionsmitteln in die Deoxyform überführen.

Ganz besonders bevorzugt ist die Ausführungsform des erfindungsgemässen Verfahrens, bei der das in der hämolysierten Blutprobe enthaltene Hämoglobin vollständig mit einem Reduktionsmittel, beispielsweise Natriumdithionit, in die Deoxyform übergeführt, mit einem oder mehreren Substanzen mit Bindungswirkung auf die allosterische Effektorstelle und/oder mit einem oder mehreren hämbindenden Liganden versetzt und danach mit Haptoglobin in Kontakt gebracht wird. Auf diese Weise wird erreicht, dass nur der glykosilierte Anteil am Gesamthämoglobingehalt einer Probe von dem Haptoglobin gebunden wird und quantitativ bestimmt werden kann.

Das Haptoglobin wird in mindestens äquimolarer Menge bezogen auf das Hämoglobin eingesetzt. Von Vorteil ist jedoch auch hier ein wesentlicher Überschuss. Vorzugsweise wird die 2- bis 50fache molare Menge verwendet.

Das Haptoglobin kann in freier Form mit der Messlösung in Kontakt gebracht werden. In diesem Falle wird zweckmässig der glykosilierte Anteil am Gesamthämoglobin in homogener Phase nach bekannten Methoden bestimmt. Beispielsweise kann der glykosilierte Anteil nach der Fluoreszenzlöschungsmethode nach Nagel und Gibson, „J. Biol. Chem.", *242* [1967], S. 3428 gemessen werden. Eine weitere Möglichkeit ergibt sich aus der unterschiedlichen pH-Abhängigkeit der Pseudoperoxidase-Aktivität von freien und haptoglobingebundenem Hämoglobin (vgl. Kawamura u. a., „Biochim. Biophys. Acta" *285* [1972], S. 22-27).

Da das Haptoglobin als ein natürlicher Antikörper für Hämoglobin angesehen werden kann, kommen als weitere Methoden zur Bestimmung der Wechselwirkung zwischen Haptoglobin und Hämoglobin sämtliche aus der Immundiagnostik bekannten Verfahren, wie Radioimmunoassay, Enzymimmunoassay u. ä., in Frage.

Es ist ferner möglich, das Haptoglobin auf einem Träger zu fixieren. In diesem Fall kann das Bestimmungsverfahren durchgeführt werden, indem man entweder

a) den mit dem Haptoglobin versehenen Träger in die hämolysierte, gegebenenfalls vorbehandelte Blutprobe eintaucht oder

b) eine definierte Menge der vorbehandelten Blutprobe auf den Haptoglobinträger auftropft oder

c) mit einer definierten Menge der vorbehandelten Blutprobe das Haptoglobin vom Träger eluiert.

Als Trägermaterialien eignen sich die für analytische Nachweisreagentien üblichen Träger, wie Papier, Zellulose, Faservliese, poröse Kunststoffe und dergleichen. Die Herstellung erfolgt durch Eintauchen in oder Besprühen mit einer haptoglobinhaltigen Lösung.

Das Haptoglobin kann auch an einen unlöslichen Träger kovalent gebunden werden. Als Träger eignen sich alle üblichen, zur Fixierung von Proteinen geeigneten Trägermaterialien. Die Verknüpfung des Haptoglobins mit dem Trägermaterial erfolgt in einer dem Fachmann allgemein bekannten Weise. Das trägerfixierte Haptoglobin wird der hämolysierten Blutprobe, die gegebenenfalls mit den oben erwähnten Zusatzstoffen versehen worden ist, zugegeben. Nach ausreichender Kontaktzeit, z. B. nach ca. 1 min, wird das unlöslich gemachte Haptoglobin mit dem gebundenen glykosilierten Hämoglobinanteil abgetrennt und das glykosilierte Hämoglobin in üblicher Weise entweder direkt photometrisch aufgrund seiner Eigenfarbe oder auch anhand seiner pseudoperoxidatischen Aktivität bestimmt.

Der haptoglobinhaltige Träger kann auch auf eine Säule gegeben werden, durch die die hämolysierte, mit eventuellen Zusätzen versehene Blutprobe hindurchgeleitet wird. Der glykosilierte Anteil des Hämoglobins bleibt bei dieser Verfahrensweise an dem trägergebundenen Haptoglobin haften und kann somit leicht von dem in Lösung verbleibenden, nicht glykosilierten Hämoglobinanteil abgetrennt werden. In diesem Falle ist es zweckmässig, den nicht gebundenen, nicht glykosilierten Hämoglobinanteil in dem Eluat zu bestimmen. Der $HbA_1$-Gehalt ergibt sich dann aus der Differenz von Gesamthämoglobin und des gemessenen, nicht glykosilierten Hämoglobinanteils.

Gegenstand der Erfindung ist ferner ein Reagens zur Bestimmung des glykosilierten Hämoglobins in einer Blutprobe, das in einem geeigneten Puffersystem ein Mittel zur Hämolyse der Erythrozyten sowie freies oder trägergebundenes Haptoglobin enthält. Auch dem Reagens können vorteilhafterweise eine oder mehrere Substanzen mit Bindungswirkung auf die allosterische Effektorstelle, eine oder mehrere hämbindende Liganden und/oder Substanzen mit stabilisierender Wirkung auf ionische Bindungen zugesetzt werden.

Ein weiterer Gegenstand der Erfindung ist ein Reagens zur Differenzierung von glykosiliertem und nicht glykosiliertem Hämoglobin, das Haptoglobin in freier oder trägergebundener Form enthält.

Die beigefügten Figuren stellen im Einzelnen dar:

Fig. 1 Abnahme der Fluoreszenzintensität einer Haptoglobinlösung nach Zusatz von glykosiliertem (Kurve 1) bzw. nicht glykosiliertem (Kurve 2) Hämoglobin.

$I_t$ = Fluoreszenzintensität zur Zeit t

$I\infty$ = Fluoreszenzintensität nach Ablauf der Reaktion

Fig. 2 Abnahme der Fluoreszenzintensität einer Haptoglobinlösung nach Zusatz von glykosiliertem Hamoglobin und Inosithexaphosphat (Kurve 1) bzw. von nicht glykosiliertem Hämoglobin und Inosithexaphosphat (Kurve 2).

$I_t$ = Fluoreszenzintensität zur Zeit t

$I\infty$ = Fluoreszenzintensität nach Ablauf der Reaktion

Fig. 3 Abhängigkeit des an Haptoglobin gebundenen Hämoglobins von dem prozentualen Anteil an glykosiliertem Hämoglobin an dem Gesamthämoglobingehalt.

Die folgenden Beispiele erläutern die Erfindung:

*Beispiel 1*

Von einer Lösung, die 0,3 mmol/l $K_3[Fe(CN)_6]$, 25 mmol/l Natriumfluorid und 45,5 mg/l Haptoglobin humanen Ursprungs (Mischtyp) in 100 mmol/l Phosphatpuffer, pH=6,70, enthält, werden 2,2 ml in eine Messküvette gegeben. Nach Zusatz von 100 µl einer 0,05%igen Lösung von glykosiliertem Hämoglobin, die ebenfalls 0,3 mmol/l $K_3[Fe(CN)_6]$ und 25 mmol/l Natriumfluorid enthält, wird die Abnahme der Fluoreszenzintensität zeitlich verfolgt (Anregungswellenlänge 280 nm, Emissionswellenlänge 330 nm). Das Messergebnis ist in Fig. 1, Kurve 1 wiedergegeben.

In gleicher Weise wie oben beschrieben wird der Verlauf der Fluoreszenzintensität gemessen, nachdem der Messlösung anstelle des glykosilierten Hämoglobins nicht glykosiliertes Hämoglobin zugesetzt worden war. Das Ergebnis ist in Fig. 1, Kurve 2 dargestellt.

*Beispiel 2*

Es werden Messlösungen vorbereitet, wie in Beispiel 1 angegeben. Anstelle des Phosphatpuffers wird ein 0,05 molarer Bis-Tris-Puffer, pH=6,70, eingesetzt [Bis-Tris-Puffer = N,N-bis-(2-hydroxyethyl)iminotris(hydroxymethyl)methan]. Vor Zugabe des glykosilierten bzw. des nicht glykosilierten Hämoglobins werden die Messlösungen mit jeweils 0,2 mmol/l Inosithexaphosphat versetzt. Die zeitliche Abnahme der Fluoreszenzintensität ist in Fig. 2 dargestellt.

*Beispiel 3*
*Herstellung von Haptoglobin-Sepharose®*

20 mg Haptoglobin humanen Ursprungs (Mischtyp) der Fa. Behringwerke AG werden an 4 g bromcyanaktivierte Sepharose® nach dem Verfahren von Klein und Mihaesco (,,Biochem. and Biophys. Research Comun.", *52* [1973] S. 774-778) gekoppelt. Durch Zugabe von 16 g inaktiver Sepharose® wird das erhaltene trägergebundene Haptoglobinpräparat verdünnt. Man erhält ein an Sepharose® gekoppeltes Haptoglobinpräparat mit einer Bindungskapazität von $5 \times 10^{-9}$ mol Hämoglobin pro 1 g Feuchtmasse.

*Bestimmung des an Haptoglobin-Sepharose® gebundenen Hämoglobins*

1 ml einer $8 \times 10^{-7}$ mol/l Lösung des Hämoglobins in 0,05 mol/l Bis-Tris-Puffers, pH 6,70, wird zur Überführung in die Deoxyform mit ca. 5 mg Natriumdithionit versetzt. Es werden nacheinander Inosithexaphosphat und N-Butylisocyanid in den folgenden Konzentrationen zugegeben.

| Probe Nr. | Hämoglobin | Inosithexaphosphat (mol/l) | n-Butylisocyanid (mol/l) | Extinktion (mE) |
|---|---|---|---|---|
| 1 | $HbA_1$ | $5 \times 10^{-5}$ | 0 | 0 |
| 2 | $HbA_1$ | $5 \times 10^{-5}$ | $5 \times 10^{-4}$ | 188 |
| 3 | $HbA_1$ | 0 | $5 \times 10^{-4}$ | 194 |
| 4 | $HbA_0$ | $5 \times 10^{-5}$ | 0 | 0 |
| 5 | $HbA_0$ | $5 \times 10^{-5}$ | $5 \times 10^{-4}$ | 0 |
| 6 | $HbA_0$ | 0 | $5 \times 10^{-4}$ | 184 |

Die Lösung wird mit 160 mg des in oben beschriebener Weise hergestellten Haptoglobin-Sepharose®-Präparates 1 min unter Schütteln intensiv vermischt. Anschliessend wird mit dithionithaltigem Puffer, zweimal nur mit Puffer gewaschen und vom Überstand abgetrennt.

Das an die Haptoglobin-Sepharose® gebundene Hämoglobin wird anhand seiner Pseudoperoxidaseaktivität mit Guajakol in bekannter Weise bestimmt. Dazu wird zur zentrifugierten Haptoglobin-Sepharose® 1 ml einer 30 mmol/l Guajakollösung in 0,1 mol/l Acetatpuffer, pH 4,0, gegeben. Durch weitere Zugabe von 50 µl einer 1%igen Wasserstoffperoxidlösung wird die Reaktion gestartet. Nach 5 min Reaktionszeit wird von der Sepharose® abzentrifugiert und die Extinktion des entstandenen Farbstoffes im Überstand bei 436 nm gemessen. Die gefundenen Extinktionswerte sind in der oben wiedergegebenen Zusammenstellung angegeben.

Die gefundenen Extinktionswerte zeigen, dass in Gegenwart von Inosithexaphosphat alleine weder glykosiliertes noch nicht glykosiliertes Hämoglobin von der Haptoglobin-Sepharose® gebunden werden. In Gegenwart von N-Butylisocyanid ohne Zusatz von Inosithexaphosphat findet eine Wechselwirkung zwischen Haptoglobin und sowohl dem nicht glykosilierten als auch dem glykosilierten Hämoglobin statt. Eine Unterscheidung zwischen nicht glykosiliertem und glykosiliertem Hämoglobin ist mit sehr guter Genauigkeit dann möglich, wenn Inosithexaphosphat und N-Butylisocyanid der Probe zugegeben werden. Die oben aufgeführten Extinktionswerte zeigen, dass in diesem Falle nur der glykosilierte Anteil des Hämoglobins an die Haptoglobin-Sepharose® gebunden wird.

*Beispiel 4*

1 ml einer $8 \times 10^{-7}$ mol/l Lösung des Hämoglobins in 0,05 mol/l Bis-Tris-Puffer, pH 6,70, wird zur Überführung in die Deoxyform mit ca. 5 mg Natriumdithionit versetzt. Es werden 25 µl einer 0,1 mol/l Lösung von Natriumnitrit zugegeben. Nach einer Reaktionszeit von 5 min wird Inosithexaphosphat in den folgenden Konzentrationen zugegeben.

| Probe Nr. | Hämoglobin | Inosithexaphosphat (mol/l) | Extinktion (mE) |
|---|---|---|---|
| 1 | $HbA_1$ | 0 | 224 |
| 2 | $HbA_1$ | $5 \times 10^{-5}$ | 190 |
| 3 | $HbA_0$ | 0 | 219 |
| 4 | $HbA_0$ | $5 \times 10^{-5}$ | 16 |

Die Lösung wird mit 160 mg eines Haptoglobin-Sepharose®-Präparates, das, wie in Beispiel 3 angegeben, hergestellt worden ist, 1 min unter Schütteln intensiv vermischt und in gleicher Weise, wie in Beispiel 3 angegeben, weiterbehandelt.

Der an die Haptoglobin-Sepharose® gebundene Hämoglobinanteil wird, wie in Beispiel 3 angegeben, bestimmt. Die gefundenen Extinktionswerte sind in der obigen Zusammenstellung angegeben.

Die gefundenen Extinktionswerte zeigen, dass in Gegenwart von Inosithexaphosphat das glykosilierte Hämoglobin in weit stärkerem Masse an die Haptoglobin-Sepharose® gebunden worden ist als die nicht glykolisierte Hämoglobinform.

*Beispiel 5*

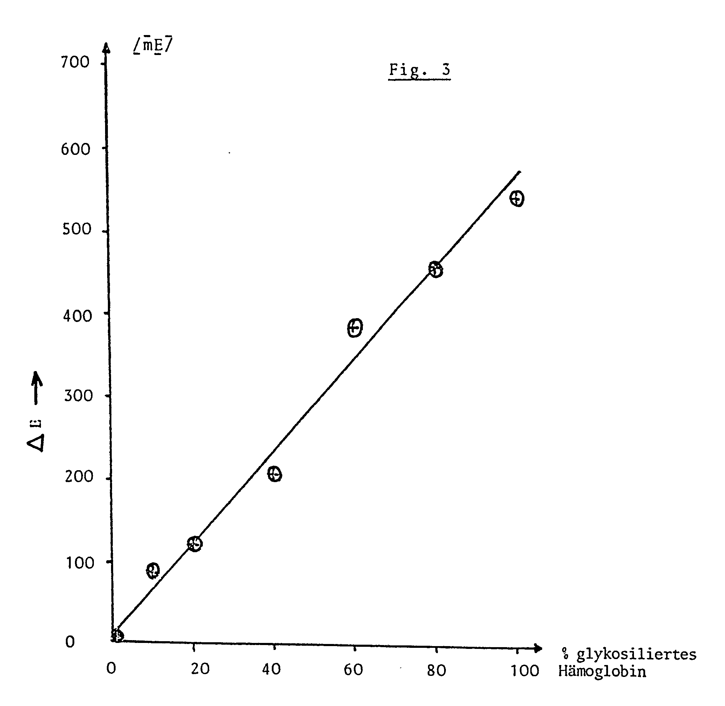

In der in Beispiel 3 angegebenen Weise werden hämoglobinhaltige Proben hergestellt, wobei der Anteil an glykolisiertem Hämoglobin von 0 bis 100 % steigt. Die Proben werden jeweils mit 5 mg Natriumdithionit, $5 \times 10^{-5}$ mol/l Inosithexaphosphat und $5 \times 10^{-4}$ mol/l N-Butylisocyanid versetzt und wie in Beispiel 3 angegeben weiter behandelt. Als Haptoglobinreagens wird ein Haptoglobin-Sepharose®-Präparat verwendet, das in analoger Weise wie in Beispiel 3 angegeben erhalten worden ist, das jedoch eine Bindungskapazität von $1,7 \times 10^{-8}$ mol Hämoglobin pro 1 g Präparat aufweist. In Fig. 3 sind die gefundenen Extinktionswerte in Abhängigkeit von dem prozentualen Anteil vom glykosilierten Hämoglobin am Gesamthämoglobingehalt aufgetragen.

Mit Hilfe der in Fig. 3 wiedergegebenen Standardkurve lässt sich der unbekannte Gehalt an glykosiliertem Hämoglobin in einer Probe nach der hier angegebenen Verfahrensweise bestimmen.

**Patentansprüche**

1. Verfahren zur Bestimmung von glykosiliertem Hämoglobin in Blutproben, wobei zunächst durch chemische oder physikalische Behandlung der Blutprobe glykosiliertes oder nicht glykosiliertes Hämoglobin aus den Erythrozyten freigesetzt, gegebenfalls das Hämoglobin in Methämoglobin übergeführt, danach eine Differenzierung von glykosiliertem und nicht glykosiliertem Hämoglobin durchgeführt und anschliessend der glykosilierte Anteil des Hämoglobins bestimmt wird, dadurch gekennzeichnet, dass die Differenzierung von glykosiliertem und nicht glykosiliertem Hämoglobin mit Hilfe von Haptoglobin vorgenommen wird.

2. Verfahren gemäss Anspruch 1, dadurch gekennzeichnet, dass die Differenzierung von glykosiliertem und nicht glykosiliertem Hämoglobin in Gegenwart eines geeigneten Puffersystems und/oder einer oder mehrerer Substanzen durchgeführt wird, die im glykosilierten und/oder nicht glykosilierten Hämoglobin eine Konformationsänderung bewirken.

3. Verfahren gemäss Anspruch 2, dadurch gekennzeichnet, dass als Substanzen, die im glykosilierten und/oder nicht glykosilierten Hämoglobin eine Komformationsänderung bewirken, Verbindungen mit Bindungswirkung auf die allosterische Effektorstelle und/oder hämbindende Liganden verwendet werden.

4. Verfahren gemäss Anspruch 3, dadurch gekennzeichnet, dass als Verbindungen mit Bindungswirkung auf die allosterische Effektorstelle Inosithexaphosphat, 2,3-Diphosphoglycerat oder Mellitsäure eingesetzt werden.

5. Verfahren gemäss Anspruch 3, dadurch gekennzeichnet, dass als hämbindende Liganden Alkylisocyanide, Sauerstoff, Kohlenmonoxid oder Stickstoffmonoxid sowie Fluorid, Azid, Cyanid oder Wasser eingesetzt werden.

6. Verfahren gemäss einem der Ansprüche 1 bis 5, dadurch gekennzeichnet, dass das Haptoglobin in 2- bis 50fachem Überschuss gegenüber dem Hämoglobin vorliegt.

7. Reagens zur Bestimmung von glykosiliertem Hämoglobin in Blutproben, enthaltend ein Mittel zur Hämolyse der Erythrozyten, ein geeignetes Puffersystem und freies oder trägergebundenes Haptoglobin sowie gegebenenfalls eine oder mehrere Substanzen mit Bindungswirkung auf die allosterische Effektorstelle, ein oder mehrere hämbindende Liganden und/oder Substanzen mit stabilisierender Wirkung auf ionische Bindungen.

8. Reagens zur Differenzierung von glykosiliertem und nicht glykosiliertem Hämoglobin, enthaltend Haptoglobin in freier oder trägergebundener Form.

**Claims**

1. Process for the determination of glycosilated haemoglobin in blood samples, whereby glycosilated or non-glycosilated haemoglobin is first liberated from the erythrocytes by chemical or physical treatment of the blood sample, the haemoglobin is optionally converted into methaemoglobin, thereafter a differentiation of glycosilated and non-glycosilated haemoglobin is carried out and subsequently the glycosilated portion of the haemoglobin is determined, characterised in that the differentiation of glycosilated and non-glycosilated haemoglobin is carried out with the help of haptoglobin.

2. Process according to Claim 1, characterised in that the differentiation of glycosilated and non-glycosilated haemoglobin is carried out in the presence of an appropriate buffer system and/or of one or more substances which bring about a conformation change in the glycosilated and/or non-glycosilated haemoglobin.

3. Process according to Claim 2, characterised in that, as substances which bring about a conformation change in the glycosilated and/or non-glycosilated haemoglobin, there are used compounds with binding action on the allosteric effector places and/or haem-binding ligands.

4. Process according to Claim 3, characterised in that, as compounds with binding action on the allosteric effector places, there are used inositol hexaphosphate, 2,3-diphosphoglycerate or mellitic acid.

5. Process according to Claim 3, characterised in that, as haem-binding ligands, there are used alkyl isocyanides, oxygen, carbon monoxide or nitrogen monoxide, as well as fluoride, azide, cyanide or water.

6. Process according to one of Claims 1 to 5, characterised in that the haptoglobin is present in 2- to 50fold excess, referred to the haemoglobin.

7. Reagent for the determination of glycosilated haemoglobin in blood samples, containing an agent for the haemolysis of the erythrocytes, a suitable buffer system and free or carrier-bound haptoglobin, as well as possibly one or more substances with binding action on the allosteric effector places, one or more haem-binding ligands and/or substances with stabilising action on ionic bonds.

8. Reagent for the differentiation of glycosilated and non-glycosilated haemoglobin, containing haptoglobin in free or carrier-bound form.

**Revendications**

1. Procédé de dosage d'hémoglobine glycosylée dans des échantillons sanguins, dans lequel on libère d'abord, au moyen d'un traitement chimique ou physique de l'échantillon sanguin, l'hémoglobine glycolysée ou non glycolysée à partir des érythrocytes, on transforme éventuellement l'hémoglobine en méthémoglobine, on effectue ensuite une différenciation d'hémoglobine glycosylée et d'hémoglobine non glycosylée, après quoi on dose la fraction glycosylée de l'hémoglobine, caractérisé en ce que la différenciation de l'hémoglobine glycosylée et de l'hémoglobine non glycosylée est effectuée à l'aide d'haptoglobine.

2. Procédé selon la revendication 1, caractérisé en ce que la différenciation entre hémoglobine glycosylée et hémoglobine non glycosylée est effectuée en présence d'un système-tampon approprié et/ou d'une ou de plusieurs substances qui provoquent une modification de la conformation de l'hémoglobine glycosylée et/ou non glycosylée.

3. Procédé selon la revendication 2, caractérisé en ce qu'on utilise, comme substances provoquant une modification de la conformation de l'hémoglobine glycosylée et/ou non glycosylée, des composés ayant un effet de liaison sur la position réactionnelle allostérique et/ou des ligands liant l'hème.

4. Procédé selon la revendication 3, caractérisé en ce qu'on utilise, comme composés ayant un effet de liaison sur la position réactionnelle allostérique, l'hexaphosphate d'inositol, le 2,3-diphosphoglycérate ou l'acide mellitique.

5. Procédé selon la revendication 3, caractérisé en ce qu'on utilise, comme ligands liant l'hème, les cyanures d'alcalis, l'oxygène, l'oxyde de carbone, l'oxyde azoteux, ainsi que le fluorure, l'azoture, le cyanure ou l'eau.

6. Procédé selon l'une des revendications 1 à 5, caractérisé en ce que l'haptoglobine est présente en un excès de 2 à 50 fois la quantité d'hémoglobine.

7. Réactif pour le dosage d'hémoglobine glycosylée dans des échantillons sanguins, contenant un agent pour l'hémolyse des érythrocytes, un système-tampon approprié et de l'haptoglobine libre ou fixée sur un support, ainsi qu'éventuellement une ou plusieurs substances ayant un effet de liaison sur la position réactionnelle allostérique, un ou plusieurs ligands liant l'hème et/ou des substances ayant un effet stabilisant sur les liaisons ioniques.

8. Réactif pour la différenciation entre hémoglobine glycosylée et hémoglobine non glycosylée, contenant l'haptoglobine sous forme libre ou fixée sur un support.

Fig. 1

**Fig. 2**

Fig. 3